# EUROPEAN PATENT APPLICATION

(11) **EP 4 420 652 A1**
(43) Date of publication of application: **28.08.2024**
(21) Application number: 24156901.1
(22) Date of filing: 09.02.2024
(51) Int. Cl.: A61K 8/06, A61K 8/31, A61K 8/68, A61K 8/891, A61K 8/893, A61Q 19/00

(54) **WATER-IN-SILICONE COSMETIC COMPOSITION INCLUDING SUBSTANCES INSOLUBLE IN WATER OR SILICONE**

(30) Priority: 27.02.2023 KR 20230025831
(71) Applicant: Amorepacific Corporation, Seoul 04386 (KR)
(72) Inventor: Cho, Youngsuk, 17074 Yongin (KR); Hwang, Yoonkyun, 17074 Yongin (KR); Park, Sungshim, 17074 Yongin (KR)
(74) Representative: Schön, Christoph

(57) **Abstract**

The present disclosure relates to a water-in-silicone cosmetic composition containing a water- or silicone-poorly soluble substance. Specifically, the present disclosure provides a water-in-silicone cosmetic composition that can contain a high content of a substance poorly soluble in water or silicone by stably dispersing it in a silicone phase or aqueous phase, and has excellent long-term stability.

## Description

### BACKGROUND OF THE INVENTION

### Field of the Invention

Disclosed herein is a water-in-silicone cosmetic composition containing a high content of a substance that is poorly soluble in water or silicone.

### Description of the Related Art

Recently, interest in high-efficacy cosmetic compositions with various effects is increasing, and in line with this trend, research on methods for stabilizing a poorly soluble effective raw material is being actively conducted.

There are various methods commonly used to stabilize a poorly soluble component, such as micro-emulsification containing hydrogenated lecithin and anionic surfactant as active ingredients, a method of dissolving the poorly soluble component in polyhydric alcohol at high temperature and dispersing them in an aqueous phase, or a method of stabilizing the components by producing alpha gel using fatty acids.

However, these methods are specialized for oil-in-water (O/W) formulations, and in these oil-in-water (O/W) compositions, ceramide precipitates over time, causing a decrease in appearance quality, which has been pointed out as a representative problem.

Therefore, there is a need to develop a composition capable of stabilizing effective raw materials in an oil phase or aqueous phase, such as a water-in-oil (W/O) or water-in-silicone (W/S) composition.

### SUMMARY OF THE INVENTION

### Technical Problem

In one aspect, the present invention is to provide a water-in-silicone cosmetic composition that can contain a high content of a substance poorly soluble in water or silicone by stably dispersing it in a silicone phase or aqueous phase, and has excellent long-term stability.

### Technical Solution

In one aspect, the present invention provides a water-in-silicone cosmetic composition including: a silicone part containing a silicone and a hydrocarbon-based oil with a weight average molecular weight of 1,000 or less; and an aqueous phase part containing water, wherein the water-in-silicone cosmetic composition comprises a poorly soluble component having a solubility in the aqueous phase part of 1 mg/ml or less throughout the water-in-silicone cosmetic composition.

### Advantageous Effects

In one aspect, the cosmetic composition disclosed herein disperses water- or silicone-poorly soluble substances in crystalline form throughout the W/S composition, whereby the final product can have excellent appearance quality and contain a high content of the poorly soluble substances.

### BRIEF DESCRIPTION OF THE DRAWINGS

FIGS. 1A and 1B are polarization images of a water-in-silicone (W/S) composition according to an embodiment of the present invention, respectively.
FIG. 2 illustrates DSC data of a water-in-silicone (W/S) composition according to an embodiment of the present invention.
FIGS. 3A and 3B are a polarization image (3A) and an scanning electron microscope (SEM) image (3B) of a water-in-silicone (W/S) composition according to an embodiment of the present invention, respectively.
FIGS. 4A to 4C show an optical image (4A), a polarization image (4B), and an appearance image (4C) observed at 40x magnification of a sample one year after production of a water-in-silicone (W/S) composition according to an embodiment of the present invention.

### DETAILED DESCRIPTION OF THE INVENTION

Hereinafter, the present invention will be described in more detail by way of the following examples. However, the following examples are provided only for illustrative purposes to aid understanding of the present invention, and the scope and range of the present invention are not limited thereto.

In exemplary embodiments of the present invention, provided is a water-in-silicone cosmetic composition including: a silicone part containing a silicone and a hydrocarbon-based oil with a weight average molecular weight of 1,000 or less; and an aqueous phase part containing water, wherein the water-in-silicone cosmetic composition comprises a poorly soluble component having a solubility in the aqueous phase part of 1 mg/ml or less throughout the water-in-silicone cosmetic composition.

In the present disclosure, a basic water-in-silicone (W/S) composition is prepared at room temperature, then the temperature of the prepared W/S contents is raised, and hydrocarbon-based oil and ceramide are dissolved in advance and slowly transferred to the already prepared W/S contents, whereby the hydrocarbon-based oil having good compatibility with silicone is stabilized within the W/S composition, and highly crystalline silicone-poorly soluble component goes through a process of being finely dispersed and precipitated throughout the W/S composition, including the silicone part and aqueous phase part. Thus, the poorly soluble component can be stabilized within the entire W/S composition.

The solubility of the poorly soluble component in the aqueous phase part may be, for example, 1 mg/ml or less, 0.1 mg/ml or less, 0.05 mg/ml or less, 0.01 mg/ml or less, or 0.001 mg/ml or less.

The weight average molecular weight of the hydrocarbon-based oil may be, for example, 1,000 or less, 900 or less, 800 or less, 700 or less, 600 or less, 500 or less, 400 or less, 300 or less, 200 or less, or 100 or less, and 100 or more, 200 or more, 300 or less, 400 or more, 500 or more, 600 or more, 700 or more, 800 or more, or 900 or more. When the weight average molecular weight of the hydrocarbon-based oil is greater than 1,000, it has poor compatibility with silicone oil, so it may be difficult to stabilize the oil particles in silicone used as the main surfactant in silicone part, which may result in separation of the contents.

In one embodiment, the hydrocarbon-based oil may have carbon atoms of 40 or less. For example, the carbon number may be 40 or less, 35 or less, 30 or less, 25 or less, 20 or less, or 15 or less.

In one embodiment, the hydrocarbon-based oil may include one or more selected from the group consisting of octane, nonane, decane, undecane, dodecane, tridecane, tetradecane, pentadecane, hexadecane, heptadecane, octadecane, vaseline, paraffin, ceresin, and squalane.

For example, the hydrocarbon-based oil may include one or more selected from the group consisting of n-octane, isooctane, n-nonane, isononane, n-decane, isodecane, n-undecane, isoundecane, n-dodecane, isosodecane, n-tridecane, isotridecane, n-tetradecane, isotetradecane, n-pentadecane, isopentadecane, n-hexadecane, isohexadecane, n-heptadecane, isoheptadecane, n-octadecane, isooctadecane, vaseline, paraffin, ceresin, and squalane.

In one embodiment, the poorly soluble component may be dispersed in a crystalline phase throughout the water-in-silicone cosmetic composition. Therefore, appearance quality according to a change in poorly soluble components over time can be improved, long-term stability can be excellent, and stability can be maintained even when a high content of an active material that is easy to precipitate is included.

In one embodiment, the crystalline phase does not exist in the form of spherical particles, but may precipitate in the form of skein or needle as confirmed in an optical microscope image.

The crystalline phase may have, for example, a size of 30 µm or less, 25 µm or less, 20 µm or less, 15 µm or less, 10 µm or less, or 5 µm or less.

When a large amount of hydrocarbon-based oil is blended, the precipitated particles may have a size of about 20 µm or more, which may cause a sensation of foreign material or change in appearance quality.

In one embodiment, the poorly soluble component may be one or more of thymol trimethoxycinnamate, cozylmethylenedioxycinnamate, asiaticoside*madecassic acid*asiatic acid, hydroxypropyl bispalmitamide MEA, hydroxypropyl bislauramid MEA, ceramide, and centella.

In one embodiment, the ceramide may be one or more selected from the group consisting of ceramide EOP, ceramide NS, ceramide NP, ceramide AS, ceramide EOS, ceramide NDS, ceramide AP, glucosylceramide, and omega hydroxy ceramide.

In one embodiment, the content of the poorly soluble component may be 1 to 10% by weight based on the total weight of the cosmetic composition. If the content of the poorly soluble component exceeds 10% by weight, the utilization may be reduced in terms of the unit cost of the effective ingredient or the feeling of use.

The content of the poorly soluble component may be, for example, 1% by weight or more, 2% by weight or more, 3% by weight or more, 4% by weight or more, 5% by weight or more, 6% by weight or more, 7% by weight or more, 8% by weight or more, or 9% by weight or more, and 10% by weight or less, 9% by weight or less, 8% by weight or less, 7% by weight or less, 6% by weight or less, 5% by weight or less, 4% by weight or less, 3% by weight or less, or 2% by weight or less, based on the total weight of the cosmetic composition.

In one embodiment, the weight ratio of the hydrocarbon-based oil to the poorly soluble component may be 1:0.5 to 10. If the weight ratio is more than 1:10, the crystallization state may be heterogeneous, or the size of the precipitated and aggregated crystalline phase may become excessive.

The weight ratio of the hydrocarbon-based oil to the poorly soluble component may be, for example, 1:0.5 or more, 1:1 or more, 1:2 or more, 1:3 or more, 1:4 or more, 1:5 or more, 1:6 or more, 1:7 or more, 1:8 or more, or 1:9 or more, and 1: 10 or less, 1:9 or less, 1:8 or less, 1:7 or less, 1:6 or less, 1:5 or less, 1:4 or less, 1: 3 or less, 1: 2 or less, 1:1 or less.

In one embodiment, the aqueous phase part may further include polyol. For example, the poorly soluble component such as ceramide NP (m.p. 98-110°C), which has a skin barrier improvement effect, has a relatively high melting point, but can be completely dissolved around 85°C when used together with a certain amount of polyol and hydrocarbon-based oil.

When preparing the above-described W/S composition, the polyol can be dissolved together with the hydrocarbon-based oil and the poorly soluble component and transferred to the silicone part. In this case, if the poorly soluble component is dissolved alone in the polyol without a solvent such as the hydrocarbon-based oil and then transferred to the silicone part, the ceramide and high-melting point wax may agglomerate together, making even dispersion difficult. In addition, when post-process is carried out through a high-speed emulsification process, the hardness of the contents may become too high, and thus, separation of the contents may occur during the process of cooling while simultaneously stirring with the paddle.

In one embodiment, the polyol may include one or more selected from the group consisting of glycerin, 1,3-butylene glycol, propylene glycol, dipropylene glycol, polyethylene glycol, and sorbitol.

In one embodiment, the silicone part may further include wax.

In one embodiment, the wax may include one or more selected from the group consisting of camuba, ceracin, cetyl esters, microcrystalline wax, montan wax, earth wax, and synthetic wax.

In one embodiment, the content of the wax may be 1 to 5% by weight based on the total weight of the cosmetic composition. The content of the wax may be, for example, 1% by weight or more, 2% by weight or more, 3% by weight or more, 4% by weight or more, and 5% by weight or less, 4% by weight or less, 3% by weight or less, or 2% by weight or less. If the wax content exceeds 5% by weight, the spreadability may not be good due to severe agglomeration between the waxes.

In one embodiment, the viscosity of the cosmetic composition may be 10,000 cps or more. If the viscosity of the cosmetic composition is less than 10,000 cps, the oil may separate due to the low viscosity.

The viscosity of the cosmetic composition may be, for example, 10,000 cps or more, 10,500 cps or more, 11,000 cps or more, 11,500 cps or more, 12,000 cps or more, 12,500 cps or more, 13,000 cps or more, 13,500 cps or more, 14,000 cps or more, 14,500 cps or more, or 15,000 cps or more.

In one embodiment, the hardness of the cosmetic composition may be 80 Kgf or less. The hardness may be, for example, 80 Kgf or less as measured by SunRheometer ComPAC-100II. If the hardness of the cosmetic composition is greater than 80, water may come out due to high shear stress in a high hardness state.

In one embodiment, the cosmetic composition may be used to improve a skin barrier.

In one embodiment, the cosmetic composition may be used to reduce the amount of skin moisture evaporation.

The cosmetic composition according to an embodiment of the present invention may further include, as a carrier ingredient, aliphatic alcohol sulfate, aliphatic alcohol ether sulfate, sulfosuccinic acid monoester, isethionate, imidazolinium derivative, methyl taurate, sarcosinate, fatty acid amide ether sulfate, alkylamidobetaine, fatty alcohol, fatty acid glyceride, fatty acid diethanolamide, vegetable oil, linoline derivative, ethoxylated glycerol fatty acid ester, or the like.

The cosmetic composition according to an embodiment of the present invention may further include a component included in a functional additive in addition to the above components. The functional additive may include a component selected from the group consisting of water-soluble vitamins, oil-soluble vitamins, polymer peptides, polymer polysaccharides, sphingo lipids, and seaweed extract.

In addition, other blending components, such as oil and fat ingredients, moisturizers, emollients, organic and inorganic pigments, organic powders, ultraviolet absorbers, preservatives, disinfectants, antioxidants, plant extracts, pH adjusters, alcohols, pigments, fragrances, blood circulation promoters, cooling agents, anhydrotics, purified water, etc., may be additionally included.

In still another exemplary embodiment of the present invention, provided is a method for improving a skin barrier, comprising administering an effective amount of the above-described cosmetic composition to a subject in need thereof.

In yet another exemplary embodiment of the present invention, provided is a use of the above-described cosmetic composition for preparing a composition for improving a skin barrier.

In yet another exemplary embodiment of the present invention, provided is a non-therapeutic use of the above-described cosmetic composition for improving a skin barrier.

In still another exemplary embodiment of the present invention, provided is a method for reducing the amount of skin moisture evaporation, comprising administering an effective amount of the above-described cosmetic composition to a subject in need thereof.

In yet another exemplary embodiment of the present invention, provided is a use of the above-described cosmetic composition for preparing a composition for reducing the amount of skin moisture evaporation.

In yet another exemplary embodiment of the present invention, provided is a non-therapeutic use of the above-described cosmetic composition for reducing the amount of skin moisture evaporation.

Hereinafter, the present invention will be described in more detail by way of the following examples. However, the following examples are provided only for illustrative purposes to aid understanding of the present invention, and the scope and range of the present invention are not limited thereto.

### EXAMPLES

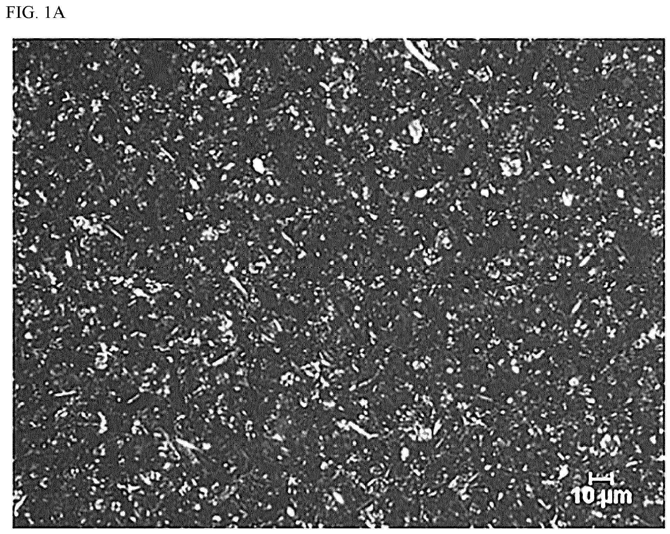

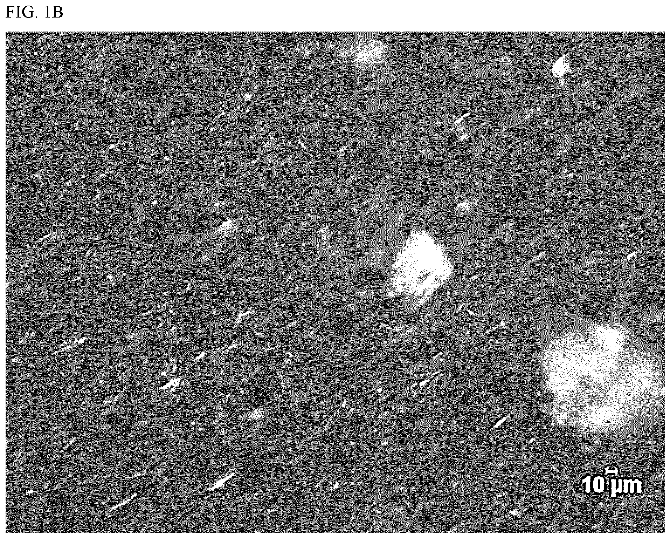

As shown in Table 1 below and FIGS. 1A and 1B, a cosmetic composition including ceramide as a poorly soluble component was prepared.

**[Table 1]**

| | Example (% by weight) | Comparative Example (% by weight) |
|---|---|---|
| Silicone | 15 | 15 |
| PEG-10 dimethicone | 1.5 | 1.5 |
| Purified water | To 100 | To 100 |
| NaCl | 1 | 1 |
| 1,2-hexandiol | 2 | 2 |
| Ethylhexylglycerin | 0.1 | 0.1 |
| Polyol* | 20 | 20 |
| Isododecane | Essential ingredient (4) | Not included |
| Synthetic wax | 3 | 3 |
| Ceramide NP | 5 | 5 |

| Formulation | W/S formulation | W/S formulation |
|---|---|---|
| Stability | O | Poor appearance quality |
| Feeling of use | Good | Sensation of foreign material confirmed |

| | | |
|---|---|---|
| Polyol*: glycerin + butylene glycol + dipropylene glycol | | |

First, in the case of the example composition, 1) silicone and PEG-silicone-based surfactant were added to a main tank and stirred, 2) low-speed homomixing was performed while slowly transferring the water part in which the purified water to the polyol part were dissolved, 3) the temperature of the prepared W/S composition was raised to 75 degrees, and thereafter, 4) isododecane, ceramide NP, and synthetic wax parts were dissolved in an external kiln and then added to the main tank while performing low-speed homomixing.

The composition of the Comparative Example was prepared in the same manner as the composition of the Example, except that isododecane, a hydrocarbon-based oil, was not included.

Hardness was measured using SunRheometer ComPAC-100II, and the compositions were observed using Nikon eclipse 80i as an optical microscope.

As a result of observation, it was confirmed that in the example composition, the ceramide was finely dispersed and precipitated in the silicone part, and was evenly dispersed and stabilized (FIG. 1A). In the case of the comparative example composition, it was confirmed that excessively large crystals were formed due to the crystallinity of ceramide, resulting in poor appearance quality and foreign material sensation (FIG. 1B). In particular, in the case of the comparative example composition, it was confirmed that the content specifications became different due to rapid precipitation in the transfer pipe during preparation. Specifically, there were differences in the hardness of cream formation and the visibility (nonconformity) of heterogeneous particles.

### Experimental Example

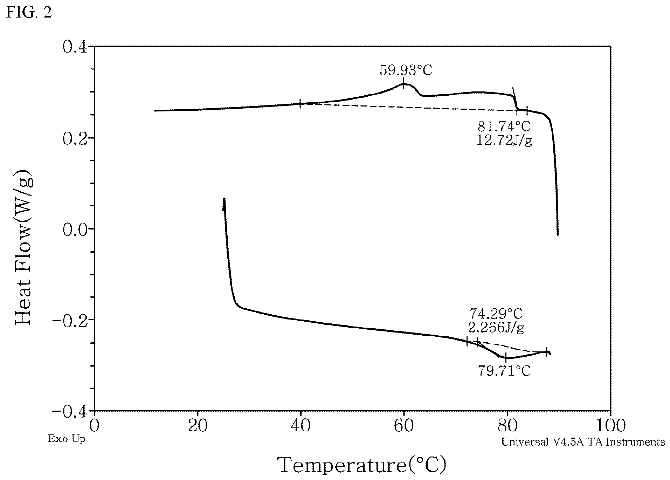

For the example compositions prepared in Table 1, DSC data was measured using a Thermal Analysis System and shown in FIG. 2. Referring to FIG. 2, it can be expected that the precipitated wax is dispersed in a stable state without affecting crystallization in the temperature range below 45°C.

If it precipitates to a size of 20-30 um or larger, the foreign matter sensation or appearance quality may change, so it is important that it is finely precipitated in a rod type without agglomeration over time.

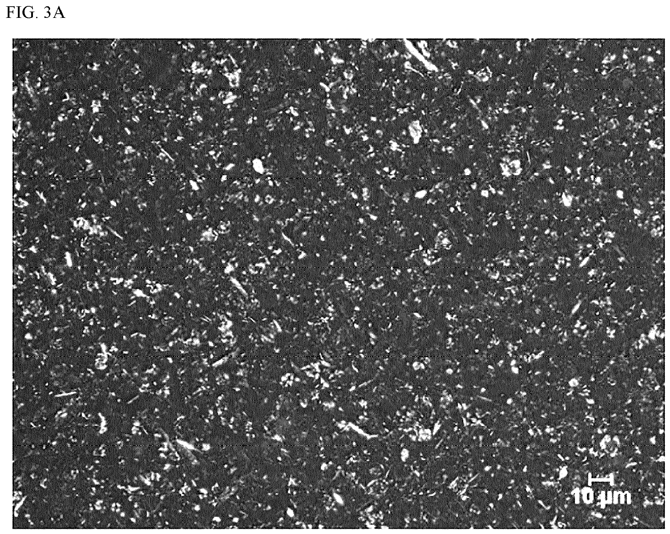

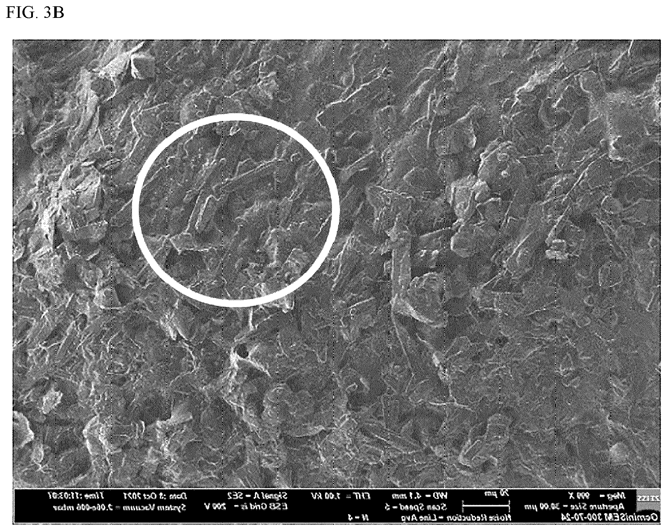

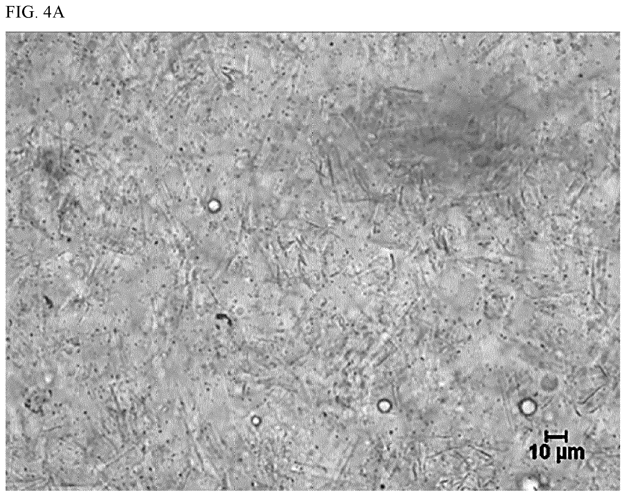

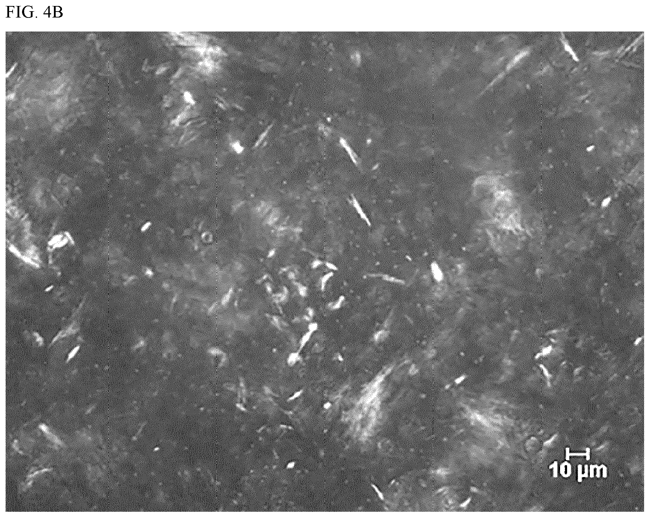

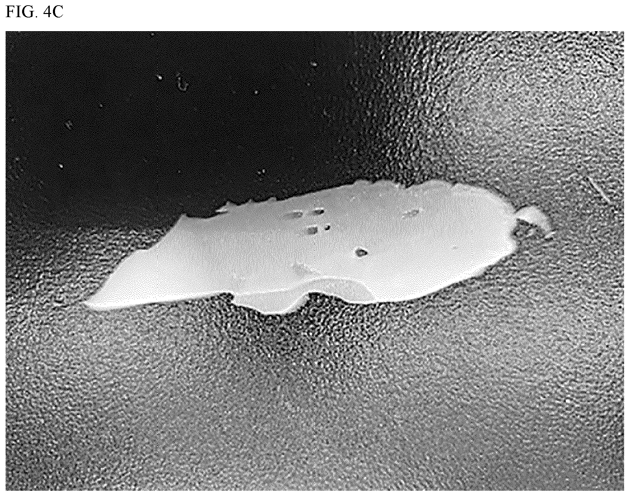

FIGS. 4A to 4C show an optical image (4A), a polarization image (4B), and an appearance image (4C) observed at 40x magnification of samples one year after the preparation of the example composition, respectively. Referring to FIGS. 4A to 4C, the issue of poor surface condition due to dissolution and crystallization of the wax part in the high temperature range was not confirmed in the samples stored for one year or more. In addition, as a result of measuring the polarization image (200 times) of the example composition prepared in Table 1, it can be confirmed that the ceramide was evenly finely dispersed as shown in FIG. 3A. As a result of observing the scanning electron microscope image (SEM) of the example composition in FIG. 3B, it can be seen that the rod-type crystallinity was clearly visible in the area highlighted by a circle.

### Embodiments

### [Embodiment 1]

A water-in-silicone cosmetic composition comprising:
a silicone part containing a silicone and a hydrocarbon-based oil with a weight average molecular weight of 1,000 or less; and
an aqueous phase part containing water,
wherein the water-in-silicone cosmetic composition comprises a poorly soluble component having a solubility in the aqueous phase part of 1 mg/ml or less throughout the water-in-silicone cosmetic composition.

### [Embodiment 2]

The water-in-silicone cosmetic composition according to embodiment 1, wherein the hydrocarbon-based oil has carbon atoms of 40 or less.

### [Embodiment 3]

The water-in-silicone cosmetic composition according to embodiments 1 or 2, wherein the hydrocarbon-based oil includes one or more selected from the group consisting of octane, nonane, decane, undecane, dodecane, tridecane, tetradecane, pentadecane, hexadecane, heptadecane, octadecane, vaseline, paraffin, ceresin, and squalane.

### [Embodiment 4]

The water-in-silicone cosmetic composition according to any one of embodiments 1 to 3, wherein the poorly soluble component is dispersed in a crystalline phase throughout the water-in-silicone cosmetic composition.

### [Embodiment 5]

The water-in-silicone cosmetic composition according to any one of embodiments 1 to 4, wherein the poorly soluble component is one or more of thymol trimethoxycinnamate, cozylmethylenedioxycinnamate, asiaticoside*madecassic acid*asiatic acid, hydroxypropyl bispalmitamide MEA, hydroxypropyl bislauramid MEA, ceramide, and centella.

### [Embodiment 6]

The water-in-silicone cosmetic composition according to embodiment 5, wherein the ceramide is one or more selected from the group consisting of ceramide EOP, ceramide NS, ceramide NP, ceramide AS, ceramide EOS, ceramide NDS, ceramide AP, glucosylceramide, and omega hydroxy ceramide.

### [Embodiment 7]

The water-in-silicone cosmetic composition according to any one of embodiments 1 to 6, wherein the content of the poorly soluble component is 1 to 10% by weight based on the total weight of the cosmetic composition.

### [Embodiment 8]

The water-in-silicone cosmetic composition according to any one of embodiments 1 to 7, wherein the weight ratio of the hydrocarbon-based oil to the poorly soluble component is 1:0.5 to 10.

### [Embodiment 9]

The water-in-silicone cosmetic composition according to any one of embodiments 1 to 8, wherein the aqueous phase part further includes polyol.

### [Embodiment 10]

The water-in-silicone cosmetic composition according to embodiment 9, wherein the polyol includes one or more selected from the group consisting of glycerin, 1,3-butylene glycol, propylene glycol, dipropylene glycol, polyethylene glycol, and sorbitol.

### [Embodiment 11]

The water-in-silicone cosmetic composition according to any one of embodiments 1 to 10, wherein the silicone part further includes wax.

### [Embodiment 12]

The water-in-silicone cosmetic composition according to embodiment 11, wherein the wax includes one or more selected from the group consisting of camuba, ceracin, cetyl esters, microcrystalline wax, montan wax, earth wax, and synthetic wax.

### [Embodiment 13]

The water-in-silicone cosmetic composition according to any one of embodiments 1 to 12, wherein the viscosity of the cosmetic composition is 10,000 cps or more.

### [Embodiment 14]

The water-in-silicone cosmetic composition according to any one of embodiments 1-13, wherein the hardness of the cosmetic composition is 80 Kgf or less.

## Claims

1. A water-in-silicone cosmetic composition comprising:
a silicone part containing a silicone and a hydrocarbon-based oil with a weight average molecular weight of 1,000 or less; and
an aqueous phase part containing water,
wherein the water-in-silicone cosmetic composition comprises a poorly soluble component having a solubility in the aqueous phase part of 1 mg/ml or less throughout the water-in-silicone cosmetic composition.

2. The water-in-silicone cosmetic composition according to claim 1, wherein the hydrocarbon-based oil has carbon atoms of 40 or less.

3. The water-in-silicone cosmetic composition according to claims 1 or 2, wherein the hydrocarbon-based oil includes one or more selected from the group consisting of octane, nonane, decane, undecane, dodecane, tridecane, tetradecane, pentadecane, hexadecane, heptadecane, octadecane, vaseline, paraffin, ceresin, and squalane.

4. The water-in-silicone cosmetic composition according to any one of claims 1 to 3, wherein the poorly soluble component is dispersed in a crystalline phase throughout the water-in-silicone cosmetic composition.

5. The water-in-silicone cosmetic composition according to any one of claims 1 to 4, wherein the poorly soluble component is one or more of thymol trimethoxycinnamate, cozylmethylenedioxycinnamate, asiaticoside*madecassic acid*asiatic acid, hydroxypropyl bispalmitamide MEA, hydroxypropyl bislauramid MEA, ceramide, and centella.

6. The water-in-silicone cosmetic composition according to claim 5, wherein the ceramide is one or more selected from the group consisting of ceramide EOP, ceramide NS, ceramide NP, ceramide AS, ceramide EOS, ceramide NDS, ceramide AP, glucosylceramide, and omega hydroxy ceramide.

7. The water-in-silicone cosmetic composition according to any one of claims 1 to 6, wherein the content of the poorly soluble component is 1 to 10% by weight based on the total weight of the cosmetic composition.

8. The water-in-silicone cosmetic composition according to any one of claims 1 to 7, wherein the weight ratio of the hydrocarbon-based oil to the poorly soluble component is 1:0.5 to 10.

9. The water-in-silicone cosmetic composition according to any one of claims 1 to 8, wherein the aqueous phase part further includes polyol.

10. The water-in-silicone cosmetic composition according to claim 9, wherein the polyol includes one or more selected from the group consisting of glycerin, 1,3-butylene glycol, propylene glycol, dipropylene glycol, polyethylene glycol, and sorbitol.

11. The water-in-silicone cosmetic composition according to any one of claims 1 to 10, wherein the silicone part further includes wax.

12. The water-in-silicone cosmetic composition according to claim 11, wherein the wax includes one or more selected from the group consisting of camuba, ceracin, cetyl esters, microcrystalline wax, montan wax, earth wax, and synthetic wax.

13. The water-in-silicone cosmetic composition according to any one of claims 1 to 12, wherein the viscosity of the cosmetic composition is 10,000 cps or more.

14. The water-in-silicone cosmetic composition according to any one of claims 1 to 13, wherein the hardness of the cosmetic composition is 80 Kgf or less.
